# EUROPÄISCHE PATENTANMELDUNG

(11) **EP 1 826 185 A2**
(43) Veröffentlichungstag der Anmeldung: **29.08.2007**
(21) Anmeldenummer: 06024107.2
(22) Anmeldetag: 21.11.2006
(51) Int. Cl.: C02F 3/28, G01N 7/18

(54) **Verfahren zur Untersuchung der biologischen Abbaubarkeit eines Stoffs**

(30) Priorität: 25.11.2005 DE 102005056561
(71) Anmelder: Universität Duisburg-Essen, 45141 Essen (DE)
(72) Erfinder: Raabe, Maren, London N19 5EE (DE); Krupp, Michaela, 45144 Essen (DE); Widmann, Renatus, 55232 Alzey (DE)
(74) Vertreter: Gesthuysen, von Rohr & Eggert

(57) **Zusammenfassung**

Es wird ein Verfahren zur Untersuchung der biologischen Abbaubarkeit eines Stoffs, insbesondere von Abfall, vorgeschlagen. Dem zu untersuchenden Stoff wird eine Bakterienkultur zugesetzt. Eine beim Abbau erfolgende Gasbildung wird erfaßt. Um feststellen zu können, ob der zu untersuchende Stoff mindestens einen unerwünschten Inhaltsstoff enthält, der den Abbau hemmt und/oder für die Bakterienkultur toxisch ist, wird dem Stoff weiter ein biologisch abbaubarer Zusatzstoff zugesetzt. Es wird zusätzlich erfaßt, ob der Zusatzstoff abgebaut wird. Wenn der Zusatzstoff abgebaut wird, liegt keine Hemmung oder toxische Wirkung durch ein unerwünschten Inhaltsstoff vor. Wenn der Zusatzstoff nicht abgebaut wird und wenn keine Gasbildung erfolgt, ist der biologische Abbau gehemmt. In diesem Fall erfolgen vorzugsweise weitergehende Untersuchungen, um über die biologische Abbaubarkeit des zu untersuchenden Stoffs endgültig entscheiden zu können.

## Beschreibung

Die vorliegende Erfindung betrifft ein Verfahren zur Untersuchung der biologischen Abbaubarkeit eines Stoffs, insbesondere von Abfall o. dgl., gemäß dem Oberbegriff des Anspruchs 1.

Das Potential der anaeroben Abbaubarkeit von Abfällen ist sowohl für die Emissionssituation auf Deponien als auch bei der Erzeugung regenerativer Energien in Biogasanlagen o. dgl. von hoher Bedeutung, Gemäß der Abfallagerungsverordnung dürfen nur noch vorbehandelte Abfälle deponiert werden. Diese Vorbehandlung kann sowohl thermisch als auch mechanisch-biologisch erfolgen. Ein Ablagerungskriterium für mechanisch-biologisch vorbehandelte Abfälle ist die Bestimmung der anaeroben Abbaubarkeit in Form der Gasbildungsrate nach 21 Tagen (GB21-Test). Eine vergleichbare oder ähnliche Bestimmung ist auch für die Charakterisierung von Stoffen für die Verwendung in der Biogasproduktion unerläßlich. Die Zahl der Biogasanlagen ist nämlich deutlich gestiegen, so daß immer mehr biogene Abfälle anaerob behandelt bzw. in Biogas umgewandelt werden.

Die vorliegende Erfindung befaßt sich mit der Untersuchung der biologischen Abbaubarkeit eines Stoffs, insbesondere von Abfällen im vorgenannten Sinne, biogenen Abfällen, Restmüll o. dgl. Insbesondere wird die biologische Abbaubarkeit - vorzugsweise unter anaeroben Bedingungen - durch Erfassung der oben genannten Gasbildungsrate nach mehreren Tagen, insbesondere 21 Tagen, untersucht bzw. bestimmt. Zur Vergleichbarkeit dieser Untersuchung bzw. Bestimmung gibt es je nach Anforderung unterschiedliche Vorschriften, z. B. die DIN 38414 S8 zur Untersuchung von Schlämmen und Sedimenten oder die Abfallablagerungsverordnung, in der die Gasbildungsrate nach einen 21 Tagen nach Zusatz einer Bakterienkultur zur Bestimmung der Ablagerungsfähigkeit von Abfällen dient. Die Vergleichbarkeit der Bestimmungen wird seit Jahren weiter entwickelt, zuletzt durch die neue VDI-Richtlinie 4630, deren Entwurf im August 2004 veröffentlicht wurde.

Trotz der genanten ständigen Weiterentwicklung ist die Untersuchung bzw. Bestimmung der biologischen Abbaubarkeit durch Erfassung der Gasbildung je nach Zusammensetzung des zu untersuchenden Stoffs problematisch. Wenn Inhaltsstoffe im zu untersuchenden Stoff hemmende oder toxische Wirkung auf die insbesondere in Form eines Impf- oder Klärschlamms zugesetzte Bakterienkultur bzw. Biozönose haben, ist der biologische Abbau gestört und es können keine repräsentativen Ergebnisse erzielt werden. Dies ist insbesondere beim Einsatz der Untersuchung der biologischen Abbaubarkeit zur Entscheidung über eine Deponierung nachteilig. So können nämlich Stoffe, die ein hohes Abbaupotential haben, minderbewertet werden, wenn die ermittelte Abbaubarkeit durch die Hemmwirkung eines (unerwünschten) Inhaltsstoffs verfälscht wird. Dies kann dazu führen, daß eigentlich nicht ablagerungsfähige Stoffe deponiert werden. Bei der Deponierung mit anderen Stoffen bzw. Abfällen kann es durch eine Verdünnung der toxischen bzw, hemmenden Inhaltsstoffe wieder zur Aufnahme von biologischen Abbauprozessen kommen. So können durch den Abbau der minderbewerteten Stoffe stark treibhauswirksame Gase, wie Methan, Geruclasemissionen und erhöhte Sickerwasserbelastungen sowie Folgekosten entstehen.

Der vorliegenden Erfindung liegt die Aufgabe zugrunde, ein verbessertes Verfahren zur Untersuchung der biologischen Abbaubarkeit eines Stoffes, insbesondere von Abfall, anzugeben.

Die obige Aufgabe wird durch ein Verfahren gemäß Anspruch 1 gelöst. Vorteilhafte Weiterbildungen sind Gegenstand der Unteransprüche.

Zur Untersuchung bzw. Bestimmung der biologischen Abbaubarkeit eines Stoffs wird diesem eine Bakterienkultur zugesetzt und eine beim biologischen Abbau erfolgende Gasbildung erfaßt. Insbesondere erfolgt die Erfassung der Gasbildung über mehrere Tage und/oder nach mehreren Tagen, vorzugsweise der Gasbildungsrate nach 21 Tagen entsprechend dem GB21-Test. Jedoch sind auch andere Bedingungen, insbesondere Zeiten, möglich.

Wie bereits erläutert, ist der Begriff "Stoff" bei der vorliegenden Erfindung sehr allgemein zu verstehen. Insbesondere handelt es sich hierbei um Abfälle, Restmüll o. dgl. Entsprechend kann es sich um einen sortenreinen Stoff, insbesondere aber auch um Gemische, Gemenge o. dgl. handeln.

Vorzugsweise liegt der zu untersuchende Stoff in flüssiger Form oder als Dispersion, Sediment, Schlamm oder als sonstiges Gemisch aus flüssigem und festem Bestandteilen vor.

Die Bakterienkultur wird dem zu untersuchenden Stoff vorzugsweise in Form eines Impf- oder Klärschlamms zugesetzt.

Zur Erfassung eines biologischen Abbaus wird die Gasbildung bzw. Gasbildungsrate erfaßt. Insbesondere wird die Bildung von Biogas, wie Kohlendioxid, Methan und/oder Spurengasen, erfaßt. Die Gasbildungsrate gibt an, wieviel Gas nach einer vorbestimmten Zeit bzw. pro Zeiteinheit pro Menge (Volumen oder Gewicht) des zu untersuchenden Stoffs gebildet wird.

Erfindungsgemäß wird dem zu untersuchenden Stoff zusätzlich zu der Bakterienkultur ein biologisch abbaubarer Zusatzstoff zugesetzt. Der Zusatzstoff kann dem zu untersuchenden Stoff zusammen mit der Bakterienkultur, davor oder danach oder zuerst der Bakterienkultur zugesetzt werden.

Der Zusatzstoff ist so gewählt, daß er von der Bakterienkultur biologisch abbaubar ist. So kann festgestellt werden, ob der zu untersuchende Stoff mindestens einen unerwünschten Inhaltsstoff enthält, der den biologischen Abbau hemmt und/oder für die Bakterienkultur toxisch ist. Dies ist nämlich dann der Fall, wenn keine oder nur eine verminderte Gasbildung erfolgt und der biologische Abbau des Zusatzstoffs gehemmt oder gar verhindert wird. In diesem Fall können dann weitergehende Untersuchungen erfolgen, um unerwünschte Inhaltsstoffe zu identifizieren und/oder die Hemmung bzw. toxische Wirkung zu analysieren und so ggf. zu verhindern, daß untersuchte Stoffe aufgrund einer durch unerwünschte Inhaltsstoffe verminderten oder gehemmten Gasbildung fälschlicherweise als nicht biologisch abbaubar eingestuft und dementsprechend deponiert werden.

Um feststellen zu können, ob der zu untersuchende Stoff mindestens einen unerwünschten Inhaltsstoff enthält, der den biologischen Abbau hemmt und/oder für die Bakterienkultur toxisch ist, wird nach Ablauf des Tests - also beispielsweise nach 21 Tagen - auch bestimmt, ob der Zusatzstoff biologisch abgebaut wurde. Dies ist grundsätzlich bzw. theoretisch durch die Erfassung der Gasbildung möglich, Wenn nämlich gar kein Gas gebildet wurde, wurde dementsprechend auch der Zusatzstoff nicht biologisch abgebaut.

In der Praxis ist der Gehalt des Zusatzstoffs jedoch vorzugsweise gering. Insbesondere beträgt die Konzentration an zugesetztem Zusatzstoff höchstens 1 g pro Liter an zu untersuchendem Substrat (Mischung aus dem zu untersuchenden Stoff und der Bakterienkultur, und ggf sonstiger Zusätze). In diesem Fall ist die Gasbildung bei biologischem Abbau des Zusatzstoffs praktisch nicht meßbar oder liegt außerhalb des Meßbereichs bzw. der Meßgenauigkeit, die zur. Erfassung der Gasbildung bei biologischem Abbau des zu untersuchenden Stoffs erforderlich ist. Vorzugsweise wird daher die Konzentration des Zusatzstoffs in dem Substrat nach Durchführung der Untersuchung zur biologischen Abbaubarkeit - also insbesondere nach 21 Tagen - bestimmt. So kann direkt, also unabhängig von der Gasbildung, festgestellt werden, ob und ggf. in welchem Umfang der Zusatzstoff abgebaut wurde.

Vorzugsweise wird die biologische Abbaubarkeit des Zusatzstoffs auch unabhängig von dem zu untersuchenden Stoff - also vorzugsweise separat und gleichzeitig - untersucht, indem eine Probe des Zusatzstoffs zusammen mit der Bakterienkultur angesetzt wird, um die tatsächliche Abbaubarkeit zu bestimmen und so durch Vergleichsergebnisse feststellen zu können, ob der biologische Abbau bei Zusatz zu dem zu untersuchenden Stoff gehemmt ist oder nicht.

Aufgrund der Vergleichswerte bzw. tatsächlichen Abbaubarkeit des Zusatzstoffs kann dann bestimmt werden, ob der dem zu untersuchenden Stoff zugesetzte Zusatzstoff im zu erwartenden Umfang - also entsprechend wie beim Vergleichstest - abgebaut wurde oder nicht. Wenn ein gleicher oder ähnlicher Abbau des Zusatzstoffs erfolgt, kann daraus gefolgert werden, daß der zu untersuchende Stoff keine unerwünschten Inhaltsstoffe enthält, die den Abbau hemmen und/oder für die Bakterienkultur toxisch sind. Entsprechend ist dann der zu untersuchende Stoff tatsächlich nicht biologisch abbaubar. Wenn hingegen der Zusatzstoff nicht oder nur vermindert biologisch abgebaut wird, zeigt dies, daß der zu untersuchende Stoff mindestens einen unerwünschten Inhaltsstoff im oben genannten Sinn enthält. In diesem Fall bieten sich dann weitergehende Untersuchungen an, um die unerwünschten Inhaltsstoffe zu identifizieren und/oder die Hemmung bzw. toxische Wirkung zu analysieren.

Vorzugsweise wird ein Zusatzstoff mit bekannter biologischer Abbaubarkeit durch die jeweilige Bakterienkultur verwendet. Besonders bevorzugt wird Phenol als Zusatzstoff verwendet. Über die bekannte biologische, insbesondere anaerobe Abbaubarkeit hinaus hat Phenol den Vorteil, daß es verhältnismäßig leicht analysierbar bzw. detektierbar ist. So kann der Gehalt bzw. die Konzentration von Phenol in dem Substrat beispielsweise durch eine einfache Analytik mittels Küvetentests und Photometer bestimmt werden. Die dazu erforderliche Ausrüstung ist im allgemeinen bei Biogasanlagen- und Deponiebetreibem bereits vorhanden, so daß lediglich ein zusätzlicher kostengünstiger Test erforderlich ist. Auch eine kostenintensive Zusatzausbildung des Betriebspersonals ist hierzu nicht erforderlich. Dementsprechend ist das vorschlagsgemäße Verfahren sehr einfach und kostengünstig durchführbar.

Das vorschlagsgemäße Verfahren wird vorzugsweise unter anaeroben Bedingungen durchgeführt. Grundsätzlich ist jedoch auch eine aerobe Durchführung bei Einsatz entsprechender Bakterien oder sonstiger Organismen möglich.

Anhand der nachfolgenden Tabelle werden verschiedene Fallkonstellationen und die daraus folgenden Schlüsse dargestellt, wobei der Begriff "Deponie" für eine zulässige oder sinnvolle Deponierung und der Begriff "Biogasanlage" beispielhaft für einen biologischen Abbau bzw. eine biologische Verwertung des Stoffs, von dem eine Probe untersucht wurde, steht:

| | Vorschlagsgemäße Untersuchung der biologischen Abbaubarkeit | | Ergebnis | Anwendungsfolge | |
|---|---|---|---|---|---|
| | Phenolabbau | Gasbildung | Bakterien | Deponie | Biogasanlage |
| 1 | Ja | Ja | aktiv | Nein | Ja |
| 2 | Ja | Nein | aktiv | Ja | Nein |
| 3 | Nein | Nein | gehemmt | Weitere Untersuchungen erforderlich | |
| 4 | Nein | Ja | selekt. gehemmt | Nein | Ja |

Im Fall 1 erfolgt ein Abbau des Phenols oder eines sonstigen Zusatzstoffs und eine Gasbildung. Dementsprechend sind die Bakterien aktiv und es kann eine Verwertung in einer Biogasanlage o. dgl. erfolgen.

Im Fall 2 erfolgt ein Phenolabbau, jedoch keine Gasbildung. Dementsprechend sind die Bakterien aktiv, der zu untersuchende Stoff ist jedoch nicht (in ausreichendem Maße) biologisch abbaubar. Folglich erfolgt in diesem Fall eine Deponierung.

Im Fall 3 erfolgt weder ein Phenolabbau noch eine Gasbildung. Die Bakterien sind also gehemmt. Hier sind weitere Untersuchungen erforderlich, um über die biologische Abbaubarkeit des zu untersuchenden Stoffs entscheiden zu können.

Im Fall 4 erfolgt kein Phenolabbau, jedoch eine Gasbildung. Auch bei dieser selektiven Hemmung kann eine Verwertung des Stoffs durch biologischen Abbau, insbesondere einer Biogasanlage o. dgl., erfolgen.

Es wird ein Verfahren zur Untersuchung der biologischen Abbaubarkeit eines Stoffs, insbesondere von Abfall, vorgeschlagen. Dem zu untersuchenden Stoff wird eine Bakterienkultur zugesetzt. Eine beim Abbau erfolgende Gasbildung wird erfaßt. Um feststellen zu können; ob der zu untersuchende Stoff mindestens einen unerwünschten Inhaltsstoff enthält, der den Abbau hemmt und/oder für die Bakterienkultur toxisch ist, wird dem Stoff weiter ein biologisch abbaubarer Zusatzstoff zugesetzt. Es wird zusätzlich erfaßt, ob der Zusatzstoff abgebaut wird. Wenn der Zusatzstoff abgebaut wird, liegt keine Hemmung oder toxische Wirkung durch einen unerwünschten Inhaltsstoff vor. Wenn der Zusatzstoff nicht abgebaut wird und wenn keine Gasbildung erfolgt, ist der biologische Abbau gehemmt. In diesem Fall erfolgen vorzugsweise weitergehende Untersuchungen, um über die biologische Abbaubarkeit des zu untersuchenden Stoffs endgültig entscheiden zu können.

Besonders bevorzugt wird der Zusatzstoff zur Standarisierung von Tests, insbesondere des GB21-Tests, zugesetzt und der Abbau des Zusatzstoffes (mit)ausgewertet.

## Patentansprüche

1. Verfahren zur Untersuchung der biologischen Abbaubarkeit eines Stoffs, insbesondere von Abfall, wobei dem Stoff eine Bakterienkultur zum Abbau des Stoffs zugesetzt wird und eine beim Abbau erfolgende Gasbildung erfaßt wird,
**dadurch gekennzeichnet,**
**daß** dem Stoff weiter ein biologisch abbaubarer Zusatzstoff zugesetzt wird, um feststellen zu können, ob der Stoff einen unerwünschten Inhaltsstoffe enthält, der den Abbau hemmt und/oder für die Bakterienkultur toxisch ist,

2. Verfahren nach Anspruch 1, **dadurch gekennzeichnet, daß** die Gasbildungsrate erfaßt wird.

3. Verfahren nach Anspruch 1 oder 2, **dadurch gekennzeichnet, daß** die Bildung von Biogas erfaßt wird.

4. Verfahren nach einem der voranstehenden Ansprüche, **dadurch gekennzeichnet, daß** die Gasbildung über mehrere Tage und/oder nach mehreren Tagen erfaßt wird.

5. Verfahren nach einem der voranstehenden Ansprüche, **dadurch gekennzeichnet, daß** die Bakterienkultur in Form eines Impf- oder Klärschlamms zugesetzt wird.

6. Verfahren nach einem der voranstehenden Ansprüche, **dadurch gekennzeichnet, daß** der Abbau anaerob erfolgt.

7. Verfahren nach einem der voranstehenden Ansprüche, **dadurch gekennzeichnet, daß** Phenol als Zusatzstoff verwendet wird.

8. Verfahren nach einem der voranstehenden Ansprüche, **dadurch gekennzeichnet, daß** die Konzentration des Zusatzstoffs höchstens 1g/l beträgt.

9. Verfahren nach einem der voranstehenden Ansprüche, **dadurch gekennzeichnet, daß** der Zusatzstoff flüssig ist.

10. Verfahren nach einem der voranstehenden Ansprüche, **dadurch gekennzeichnet, daß** ein vorzugsweise zumindest im wesentlichen flüssiges Gemisch oder Gemenge aus dem Stoff, dem Zusatzstoff, der Bakterienkultur und ggf. sonstigen Zusätzen gebildet wird, dessen Gasbildung erfaßt wird.

11. Verfahren nach einem der voranstehenden Ansprüche, **dadurch gekennzeichnet, daß** insbesondere nach Erfassung der Gasbildung überprüft wird, ob der Zusatzstoff abgebaut wurde.

12. Verfahren nach einem der voranstehenden Ansprüche, **dadurch gekennzeichnet, daß** zur Überprüfung des Abbaus des Zusatzstoffs die Konzentration des Zusatzstoffs in dem Gemisch bzw, Gemenge bestimmt und dies mit einer anfänglichen Konzentration verglichen wird.

13. Verfahren nach einem der voranstehenden Ansprüche, **dadurch gekennzeichnet, daß** die biologische Abbaubarkeit des Zusatzstoffes durch die jeweilige Bakterienkultur unabhängig vom zu untersuchenden Stoff bekannt ist oder bestimmt wird.

14. Verfahren nach einem der voranstehenden Ansprüche, **dadurch gekennzeichnet, daß** die Abbaubarkeit des Zusatzstoffes unabhängig bzw. ohne den zu untersuchenden Stoff mit der Abbaubarkeit des Zusatzstoffes in Kombination mit dem Stoff durch die Bakterienkultur verglichen wird, um feststellen zu können, ob der Stoff biologisch abbaubar ist oder nicht und/oder einen die Abbaubarkeit hemmenden Inhaltsstoff enthält, und/oder um den zu untersuchenden Stoff hinsichtlich einer Abbaubarkeit klassifizieren zu können.

15. Verfahren nach einem der voranstehenden Ansprüche, **dadurch gekennzeichnet, daß** die Gasbildung und der Abbau des Zusatzstoffes erfaßt und daraus die biologische Abbaubarkeit des Stoffes bestimmt und/oder der Stoff hinsichtlich seiner Verwertbarkeit bzw. Abbaubarkeit klassifiziert wird.

16. Verfahren nach einem der voranstehenden Ansprüche, **dadurch gekennzeichnet, daß** eine vorbestimmte Menge oder Konzentration des Zusatzstoffes dem Stoff zugesetzt wird und daß der Abbau des Zusatzstoffes als zusätzliche Information ausgewertet wird, um die biologische Abbaubarkeit bzw, Verwertbarkeit des Stoffes zu bestimmen.
